# DEMANDE DE BREVET EUROPEEN

(11) **EP 0 868 899 A1**
(43) Date de publication de la demande: **07.10.1998**
(21) Numéro de dépôt: 98400740.1
(22) Date de dépôt: 30.03.1998
(51) Int. Cl.: A61K 7/06

(54) **Utilisation d'(oxo-1 alkyl)amin-2 deoxy-2 glucopyranosides de dihydroxy-2,3 propyle pour le traitement de la chute des cheveux**

(30) Priorité: 04.04.1997 FR 9704145
(71) Demandeur: ELF ATOCHEM S.A., 92800 Puteaux, Hauts-de-Seine (FR)
(72) Inventeur: Bernard, Daniel, 92400 Courbevoie (FR); Caupin, Henri-Jean, 78000 Versialles (FR); Petit, Serge, 74540 Cusy (FR)
(74) Mandataire: Haicour, Philippe

(57) **Abrégé**

La présente invention est relative à l'utilisation de formules cosmétiques à base d'(oxo-1 alkyl) amino-2 déoxy-2 glucopyranosides de dihydro-2,3 propyle répondant à la formule générale 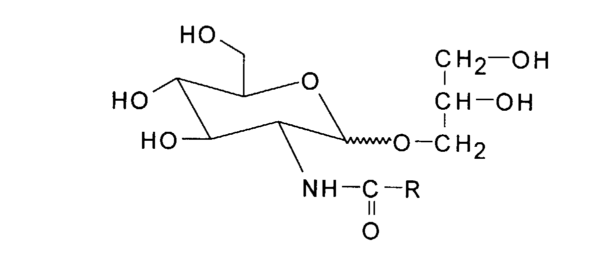 pour enrayer la chute de cheveux et en faciliter la repousse.

## Description

La présente invention a pour objet des compositions cosmétiques à usage topique destinées à induire et stimuler la croissance des cheveux et/ou freiner leur chute.

Chez l'être humain, la croissance des cheveux et leur renouvellement sont principalement déterminés par l'activité des follicules pileux. Leur activité est cyclique et comporte essentiellement trois phases, à savoir les phases anagène, catagène et télogène. A la phase anagène, active, ou phase de croissance, qui dure plusieurs années et au cours de laquelle les cheveux s'allongent, succède une phase catagène transitoire d'environ deux semaines, au cours de laquelle le follicule régresse et involue, puis une phase de repos ou phase télogène qui dure environ 70 jours, à la fin de laquelle le cheveu tombe. A la suite de quoi, un autre cycle recommence. Les follicules sont génétiquement programmés pour donner lieu à quelques 25 repousses, et un cycle normal dure en moyenne cinq ans, ce qui permet le renouvellement permanent de la chevelure au cours d'une existence. Sur les 150.000 cheveux environ que comporte une chevelure, il en tombe ainsi normalement environ 80 chaque jour, qui seront remplacés en quelques mois.

L'éclaircissement précoce de la chevelure ou alopécie qui survient chez certains sujets a une cause à la fois génétique et hormonale. Génétique, quand elle correspond au "caractère chauve" qui se transmet de père en fils, plus rarement de mère en fils, vraisemblablement porté par plusieurs gènes. Hormonale, quand elle est due à la fixation sur des récepteurs moléculaires de la racine de certains produits de dégradation enzymatique de la testostérone, la principale hormone mâle, qui se développent, par exemple à la suite d'un stress. Le résultat en est l'accumulation de collagène dans la gaine conjonctive (fibrose), entraînant le ralentissement de la croissance du cheveu, et plus tard sa mort. Les perturbations du renouvellement capillaire se traduisent dans un premier temps, par l'accélération de la fréquence des cycles aux dépens de la qualité des cheveux, puis de leur quantité : il y a appauvrissement progressif de la chevelure par régression des cheveux dits "terminaux" au stade de duvet, et finalement il arrive un moment où plus rien ne repousse. Chez l'homme les zones touchées sont plutôt les golfes temporaux ou frontaux et la zone occipitale chez l'homme. Chez la femme, on constate une alopécie diffuse du vertex.

Rien qu'en France, plus de 11 millions d'hommes sont touchés directement par la chute des cheveux. On recherche donc, depuis de nombreuses années, dans l'industrie cosmétique ou pharmaceutique, des composés et des compositions permettant de supprimer ou de réduire l'effet de l'alopécie, et notamment d'induire ou de stimuler la croissance des cheveux ou de diminuer leur chute.

Dans les approches utilisant une base carbohydrate, on a récemment proposé l'action synergique de mono-, di- et oligo-saccharides avec divers actifs; par exemple le fructose en association avec la polyvinyl pyrrolidone (voir JP08040846), le tréhalose avec du chitosan et divers actifs naturels (voir JP 08020514), les produits d'hydrolyse de l'acide alginique avec un accélérateur de la circulation sanguine et un activateur cellulaire (voir WO 9607393), l'amidon en association avec de l'oxyde de zinc et l'acide borique (voir WO 9535086), du glucose ou du fructose en association avec des protéines globulaires de céréales ou leurs hydrolysats (voir FR 2704751).

On a proposé des oligosaccharides contenant au moins une unité disaccharide constituée d'un reste uronique et d'un reste d'hexosamine (voir EP 211610).

On a proposé également des aldonolactones ou des acides hexosacchariques, par exemple la D-fucono-1,5-lactone (voir EP 531111), des formes lactames de saccharides, par exemple le L-arabino-1,5-lactame, le D-glucurono-6,3-lactame (voir EP 0334586), un polymère de cellobiono-lactone, obtenu via sa dérivation en N-p-vinyl benzyl D-cellobionamide (voir JP 05043418).

On a proposé aussi des glycosides d'ergostérol (voir JP 07101835 et JP 07109293), de stigmastérol (voir JP 07138181), de stigmastanol (voir JP 07109294), l'acide p-β-D-glucopyranosyloxy-cinnamique (voir JP 07258042) ou de glucosides dérivés de saponines (voir WO 9406402).

Mais rares sont les structures résultant du greffage d'une chaîne d'alkyle sur un polyol. On a cependant proposé le monopentadécanoyl glycérol (voir Yokoyama Daisaburo, Yukagaku 1995, 44(4) 266-73), des polyalcools-glycéryl-éthers, notamment ceux obtenus par condensation du pentaérythritol et du pentadécyl-glycidyl-éther (voir JP 08157331), et plus particulièrement, pour les structures dérivant de carbohydrates, l'undécylényl tréhalose (voir JP08053326) et des alkylpolyglycosides et/ou dérivés O-acylés du glucose (voir WO 9302657) correspondants à la structure 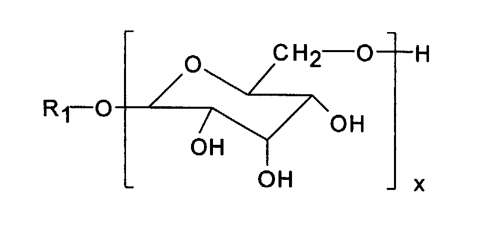 dans laquelle R₁ est un radical ou un mélange de radicaux alkyle ou alcényle et/ou à la structure 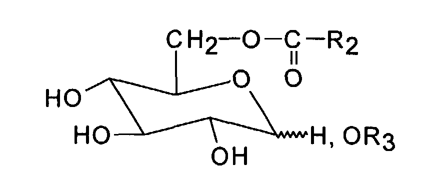 dans laquelle R₂ représente une chaîne hydrocarbonée, linéaire R₃ représente l'hydrogène ou un groupe alkyle inférieur en C₁-C₄. Toutes ces structures, sur base carbohydrate, diffèrent de celle de l'invention

On connaît aussi comme agent d'amélioration capillaire le Minoxidil® et l'Aminexil®. Le Minoxidil ou diamino-2,4-pipéridino-6-pyrimidine-3-oxyde ainsi que ses dérivés (voir brevets US 3,461,461, 3,973,061, 3,464,987 et 4,139,619), molécules connues au départ pour faire baisser la tension, se sont avérées actives dans le traitement de la chute des cheveux. Elles stimulent la division des cellules qui constituent le cheveu, favorisent la vascularisation de la racine et enfin, ralentissent la fibrose et l'atrophie de la racine du cheveu.

Mais, si le Minoxidil® ralentit la chute des cheveux chez un tiers des patients, et provoque une légère repousse chez un autre tiers, il reste sans effet sur les autres sujets traités. Appliqué matin et soir sous forme de lotion, il entraîne parfois des effets secondaires, tels que des palpitations, une baisse de tension ou de l'eczéma. Par ailleurs, les cheveux deviennent gras au cours de ce traitement généralement permanent.

L'Aminexil®, de structure proche de celle du Minoxidil® (voir WO 9609048), est le diamino-2,4-pyrimidine-3-oxyde, une molécule capable de s'opposer à la production de la principale enzyme responsable de la fibrose. Mais ce produit cosmétique n'agit malheureusement pas que les autres causes de l'alopécie que sont les facteurs hormonaux et vasculaires, ce qui limite son champ d'application à une fraction des sujets touchés par l'alopécie.

D'une manière générale, il reste intéressant et utile de pouvoir disposer de composés actifs autres que ceux déjà connus.

Les composés du type (oxo-1-alkyl)amino-2, déoxy-2, glucopyranoside de dihydroxy-2,3 propyle ont été préconisés dans des compositions lavantes pour les cheveux, avec notamment des propriétés anti-pelliculaires et de conditionnement du cheveu (voir FR 9512215), mais la demanderesse vient de découvrir de manière surprenante que ces composés permettent d'induire et de stimuler la croissance des cheveux et/ou de diminuer leur chute, chez les utilisateurs sujets à l'alopécie.

La présente invention est ainsi un procédé pour induire et de stimuler la croissance des cheveux et/ou de diminuer leur chute, qui consiste à appliquer sur les cheveux humides et sur les zones alopéciques du cuir chevelu, pendant des durées supérieures à 2 minutes et de préférence supérieures à 5 minutes, des lotions à base de (oxo-1 alkyl)amino-2, déoxy-2 glucopyranoside de dihydroxy-2,3 propyle dont la teneur en glucopyranoside. Les lotions à faibles teneur en principe actif s'appliquent sans rinçage ou avec un rinçage à l'eau après plusieurs heures. Les lotions à forte concentration sont laissées au contact quelques minutes, puis évacuées par rinçage. Les applications sont quotidiennes ou biquotidiennes, le traitement durant pour les cas moyens de trois à six mois; pour les cas plus dramatiques, sur observation du patient ou du capilliculteur, le traitement pourrait être quotidien et à vie. Ces compositions ne provoquent pas d'irritation du cuir chevelu, même après un contact prolongé sans rinçage.

Le principe actif de ces lotions est un composé répondant à la formule générale 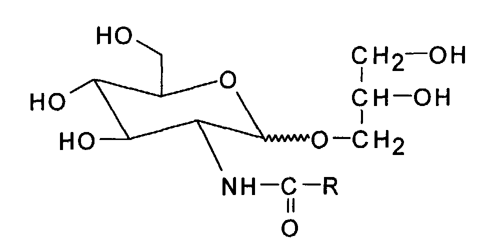 formule dans laquelle R est une chaîne hydrocarbonée linéaire ou ramifiée, saturée ou insaturée à 5 - 21 atomes de carbone, et plus particulièrement à 7 à 13 atomes de carbone. Le dérivé (oxo-1 undécényl-10)amino-2, déoxy-2 glucopyranoside de dihydroxy-2,3 propyle étant un composé préféré. La synthèse de ces composés a été décrite dans la demande de brevet français numéro 95 12215.

Les lotions utilisées à cet effet, et qui sont également des objets de l'invention, sont des compositions hydroalcooliques comportant au moins le glucopyranoside ci-dessus, le glucopyranoside entrant pour 0,1 à 30 % en poids par rapport au poids total de la composition, plus précisément pour 0,1 à 15 %, de préférence de 0,25 à 10% pour les compositions sans rinçage et pour 2 à 30 %, de préférence 3 - 15 % pour les compositions avec rinçage. Le pH de ces compositions est compris entre 3 et 9 et de préférence entre 4,5 et 7,5.

Les solvants hydroalcooliques représentent de 5 et 95 % en poids par rapport au poids total de la composition, les composants alcooliques étant des produits acceptables sur le plan cosmétique, en particulier les alcools inférieurs en C₁-C₄, le glycérol, les alcynes glycols.

Ces compositions peuvent contenir d'autres adjuvants de la cosmétique ou de la pharmacie, en vue de réaliser des compositions topiques, notamment des huiles de synthèse, des épaississants, des conservateurs, des agents alcalinisants ou acidifiants.

On réalise des lotions encore plus efficaces en associant le glucopyranoside en compositions synergiques avec d'autres composés déjà signalés pour améliorer la repousse des cheveux ou en freiner la chute, en particulier :
- le Minoxidil® ou les dérivés de pyrimidine voisins décrits dans le brevet US 4,139,619, ou
- l'Aminexil® ou les dérivés voisins pyrimidine-oxyde décrits dans le brevet WO 9609048, mais aussi
- les rétinoïdes comme l'acide trans-rétinoïque, l'isotrétinoïne, le rétinol ou vitamine A et ses dérivés, tels que l'acétate, le palmitate ou le propionate, le motrétinide, l'étrétinate, le transrétinoate de zinc
- les agents antibactériens, notamment l'érythromicine;
- les agents antagonistes du calcium, comme la Cinnarizine et le Diltrazem;
- des hormones, telles que l'oestriol ou des analogues, ou la thyroxine ou ses sels;
- des agents antiandrogènes, tels que l'oxendolone, la spironolactone;
- des inhibiteurs de 5-α-réductase;
- des capteurs de radicaux OH;
- la dyphylline et ses sels ;
- les esters d'acide nicotinique, dont notamment le nicotinate de tocophérol, le nicotinate de benzyle et les nicotinates d'alkyles en C₁-C₈ comme les nicotinates de méthyle ou d'hexyle;
- les agents anti-inflammatoires stéroïdiens et non stéroïdiens, en particulier l'hydrocortisone, ses sels et ses dérivés, l'acide niflumique et autres ;
- des esters de l'acide citrique, par exemple le citrate de tributyle.

D'autres composés peuvent être ajoutés, à savoir par exemple des sels, notamment de sodium, magnésium, calcium, zinc, cuivre, d'acides aliphatiques ou leurs mélanges, notamment d'acide propionique, butyrique, valérique, undécylénique, des esters d'acides aliphatiques, notamment de méthyl, d'éthyl, d'isopropyl, par exemple de l'acide undécylénique, des acides hydroxycarboxyliques ou cétocarboxyliques et leurs esters, l'acide salicylique et ses dérivés décrits dans le brevet français FR2581542, comme les dérivés salicyliques porteurs d'un groupement alcanoyle ayant de 2 à 12 atomes de carbone, l'acide cinnanique et ses dérivés, comme par exemple l'acide p-β-D-glucopyranosyloxy-cinnamique, des cinnamaldéhydes substitués, comme par exemple le p-méthoxycinnamaldéhyde, l'acide férulique, l'urée, des alcools terpéniques comme par exemple le thymol, des polymères comme par exemple l'amidon, le chitosan, la chitine, l'acide hyaluronique, les alginates, des protéines comme par exemple des protéines globulaires de céréales, et les produits d'hydrolyse plus ou moins avancée de ces polymères, leurs complexes avec le cuivre, des polyaminoacides comme par exemple les polyglutamates, des esters de glycérol ou polyglycérols, notamment des mono ou diesters d'acides gras de mono, di ou d'oligoglycérols, par exemple les cocoates, les laurates, les undécylénates de mono ou d'oligoglycérols, des éthers de polyalcools, notamment ceux du glycérol, des polyglycérols ou du pentaérythritol, des monomères, dimères ou oligomères de saccharides comme par exemple le glucose, le fructose, le galactose, le tréhalose, des esters de saccharides, comme par exemple l'undécényl tréhalose décrit dans JP 08053326, les dérivés O-acylés du glucose décrits dans WO 9302657, des glycosides, notamment d'ergostérol, de stigmastérol, de stigmastanol, les alkyl polyglucosides également décrits dans WO 9302657, des formes lactames de saccharides, comme par exemple le L-arabino-1,5-lactame ou le D-glucurono-6,3-lactame décrits dans EP 0334586, des lactones comme la D(L)pantolactone, des α-pyrones comme celles décrites dans le brevet EP 0672406, comme par exemple la 5-méthyl-δ-valérolactone, ou des formes lactones de saccharides, notamment celles décrites dans la demande européenne EP 531111, comme par exemple la D-fucono-1,5-lactone, des composés de type alcanamido ammonium, comme par exemple un alcanamido-triméthyl-propylammonium, un carboxyméthyl alcanamido-diméthylpropylammonium, notamment dérivé de l'acide laurique, undécylénique ou de coupes laurique/myristique, ou l'iodure de (3-(docosanamido)propyl)-triméthylammonium ou l'iodure de (3-(11-(dodécylthio)undécanamido)propyl)N,N,N-triméthylammonium.

La demanderesse a également constaté que ces composés ont une action contre le blanchiment des cheveux.

Le milieu utilisé dans ces compositions est généralement constitué de glycérol, matrice inhérente à son procédé de préparation hors purification ultime, d'eau, ou des mélanges d'eau et d'un solvant ou mélanges de solvants.

Les exemples non limitatifs suivants feront mieux comprendre l'invention. Dans ces exemples, les sujets soumis à l'expérimentation sont des volontaires, de sexe masculin, présentant une alopécie accélérée et un début de dégarnissage capillaire et n'ayant effectué aucun traitement capillaire depuis plusieurs mois.

### EXEMPLE 1:

On prépare une lotion de composition suivante :
- (oxo-1 undécényl-10) amino-2 déoxy-2 glucopyranoside de dihydroxy-2,3 propyle 5,0 g
- glycérol 20,0 g
- éthanol qsp 100,0 g
- parfum qs

On applique 2 ml de cette solution sur la chevelure humide et sur le cuir chevelu des zones touchées par l'alopécie, à la fréquence de deux fois par jour. On observe dès la 12éme semaine d'application un net ralentissement de la chute des cheveux (constaté au brossage) et dès le 6ème mois, chez plus de 30% des sujets, une repousse sur les lobes frontaux.

Remarque : on peut sans inconvénient remplacer les 100 g d'éthanol par le mélange propylène-glycol/isopropanol 20/80.

### EXEMPLE 2 :

On prépare la composition suivante :
- (oxo-1 undécényl-10) amino-2 déoxy-2 glucopyranoside de dihydroxy-2,3 propyle 10,0 g
- Glycérol 40,0 g
- ORAMIX®NS1 10,0 g
- Natrosol® 0,5 g
- Parfum qs
- Eau qsp 100,0 g

Dans cette formulation, Oramix®NS10 (SEPPIC) est un alkylpolyglucoside contenant trois unités glucose (partie hydrophile) et des chaînes en C10/C12/C14 (partie lipophile), en présentation à 55 de matière active, à fonction d'agent moussant ; Natrosol® est une cétyl- ou hexadécyl-2 hydroxyéthylcellulose, épaississant commercialisé par Aqualon.

On observe des résultats analogues à ceux décrits dans l'exemple 1; toutefois la formule permet, par un meilleur moussage, une répartition plus uniforme du produit.

### EXEMPLE 3:

On prépare la composition suivante :
- Glycérol 40,0 g
- ORAMIX®NS1 10,0 g
- Natrosol® 0,5 g
- Parfum qs
- Eau qsp 100,0 g

On n'observe aucun effet, ni sur la repoussse des cheveux, ni sur la diminution de leur chute, même après six mois d'application journalière avec 2 ml de la solution sur la chevelure humide et sur les zones touchées par l'alopécie.

### EXEMPLE 4:

On prépare la composition suivante :
- oxo-1 undécényl-10) amino-2 déoxy-2 glucopyranoside de dihydroxy-2,3 propyle 3,0 g
- glycérol 15,0 g
- cocoamphodiacétate sodique à 38% matière active 5,0 g
- acide alginique 0,5 g
- parfum qs
- eau qsp 100,0 g

(Le cocoamphodiacétate sodique est le Miranol®C2M Rhône-Poulenc); l'acide alginique est le Texamid®778 d'Henkel)

Avec cette formule, on observe des résultats analogues à ceux décrits dans l'exemple 1. La facilité d'application uniforme est la même que celle de l'exemple 2.

### EXEMPLE 5: composition mixte

On prépare la lotion de composition suivante :
- (oxo-1 undécényl-10) amino-2 déoxy-2 glucopyranoside de dihydroxy-2,3 propyle 5,0 g
- glycérol 20,0 g
- Minoxidil® 5,0 g
- éthanol qsp 100,0 g
- parfum qs

On applique 2 ml de cette solution sur la chevelure humide et les zones touchées par l'alopécie, à la fréquence de deux applications par jour. Les résultats observés sont, tant au niveau du ralentissement de la chute des cheveux qu'au niveau de la repousse sur les lobes frontaux de l'ordre de 10 à 20 % supérieurs à ceux que l'on observe dans les exemples 1 et 2.

## Revendications

1. Utilisation pour le traitement cosmétique de la chute des cheveux, d'(oxo-1-alkyl)amino-2, déoxy-2 glucopyranosides de dihydroxy-2,3 propyle, composés répondant à la formule 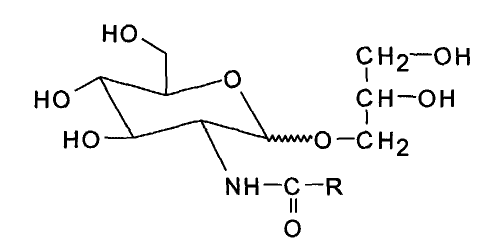 dans laquelle R représente un groupement alkyle linéaire ou ramifié, saturé ou insaturé, renfermant de 5 à 21 atomes de carbone.

2. Utilisation selon la revendication 1 d'(oxo-1-alkyl)amino-2, déoxy-2 glucopyranosides de dihydroxy-2,3 propyle, caractérisée en ce que le groupement alkyle R renferme de 7 à 17 atomes de carbone.

3. Utilisation selon la revendication 1 d'(oxo-1-alkyl)amino-2, déoxy-2 glucopyranosides de dihydroxy-2,3 propyle, caractérisée en ce que le groupement alkyle R renferme de 7 à 13 atomes de carbone.

4. Utilisation selon la revendication 1, caractérisée en ce que l'(oxo-1-alkyl)amino-2, déoxy-2 glucopyranoside de dihydroxy-2,3 propyle est l'(oxo-1 undécényl-10)amino-2, déoxy-2 glucopyranoside de dihydroxy-2,3 propyle.

5. Traitement cosmétique pour enrayer la chute des cheveux ou pour en améliorer la repousse caractérisé en ce que l'on applique pendant des durées supérieures à 2 minutes et de préférence supérieures à 5 minutes, sur les zones alopéciques du cuir chevelu et sur les cheveux humides de ces zones, des compositions hydroalcooliques contenant au moins 0,1 à 30 % d'un glucopyranoside, le glucopyranoside répondant à la formule 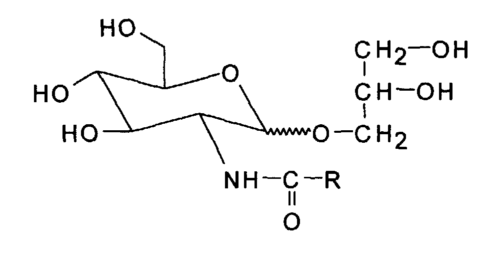 dans laquelle R représente un groupement alkyle linéaire ou ramifié, saturé ou insaturé, renfermant de 5 à 21 atomes de carbone.

6. Traitement cosmétique selon la revendication 5, dans laquelle le groupement R du glucopyranoside renferme de 7 à 17 atomes de carbone.

7. Traitement selon la revendication 5, dans laquelle l'(oxo-1-alkyl)amino-2, déoxy-2 glucopyranoside de dihydroxy-2,3 propyle est l'(oxo-1 undécényl-10)amino-2, déoxy-2 glucopyranoside de dihydroxy-2,3 propyle.

8. Composition hydroalcoolique comportant un (oxo-1-alkyl)amino-2, déoxy-2 glucopyranoside de dihydroxy-2,3 propyle, répondant à la formule 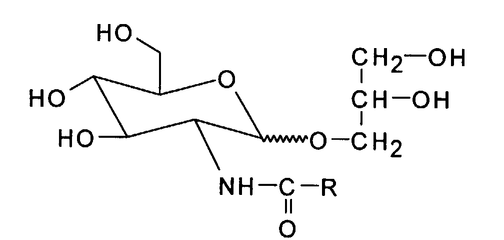 dans laquelle R représente un groupement alkyle linéaire ou ramifié, saturé ou insaturé, renfermant de 5 à 21 atomes de carbone et du diamino-2,4-pipéridino-6-pyrimidine-3-oxyde ou du diamino-2,4-pyrimidine-3-oxyde.
